# EUROPEAN PATENT SPECIFICATION

(11) **EP 0 720 842 B1**
(45) Date of publication and mention of the grant of the patent: **09.04.2003**
(21) Application number: 96300104.5
(22) Date of filing: 05.01.1996
(51) Int. Cl.: A61F 5/44

(54) **Bag for containing liquid**
Beutel zur Aufnahme einer Flüssigkeit
Poche pour la réception d'un liquide

(30) Priority: 05.01.1995 GB 9500123; 17.11.1995 GB 9523572
(43) Date of publication of application: 10.07.1996
(73) Proprietor: E.R. SQUIBB & SONS, INC., Princeton, New Jersey 08543-4328 (US)
(72) Inventor: Falconer, Malcolm I., London SW17 7DG (GB)
(74) Representative: Holmes, Miles Keeton

(56) References cited:
- DE-A- 3 722 251
- GB-A- 657 363
- US-A- 4 838 883
- US-A- 5 026 362
- US-A- 5 234 420

## Description

This invention relates to a bag for containing liquid, e.g. urine; and relates particularly though not exclusively to a leg bag and a holder therefor. Leg bags are known. They are usually bags of plastics material provided with an inlet and optionally an outlet. They are used by incontinents. Their purpose is to temporarily store urine. The urine is conducted from the bag wearer to the bag via a suitable incontinence appliance and a plastics or rubber tube.

Various methods of attaching a leg bag to the leg of the wearer have been proposed.

It is an aim of the present invention to provide a bag for containing liquid 'and holder therefor which is comfortable to wear, unobtrusive, in which changing of the bag is readily accomplished, and which includes means for ensuring that the bag can be securely held in place on the wearer.

Reference is made to US-A-5 234 420 (with respect to which the invention is characterised) and to US-A-5 026 362.

According to the invention, there is provided a combination comprising a bag for containing liquid, a holder wearable by a user, an inflow tube probe and an attachment device, wherein:
the holder comprises a first sheet of material which can conform to the curvature of a portion of the body of the wearer, a second sheet of material attached to the first sheet for movement relative to the first sheet generally about an axis;
the bag has an entry hole at its upper end into which the inflow tube probe is receivable and first and second through holes through which at least a portion of the attachment device may pass; characterised in that the attachment device is connected to the first sheet or to the inflow tube probe, and consists of a clamp which when open permits the tube probe to be inserted in or separated from the entry hole of the bag, and when closed and engaged with the through holes secures the tube probe relative to the bag to prevent removal of the tube probe.

Advantageously, the second sheet is pivotable relative to the first sheet about a substantially horizontal axis located at or near the bottom of the first sheet, though it is also possible for it to be hinged, door-like, about a substantially vertical axis.

Other features of the invention will become apparent from the following particular description, given with reference to the accompanying drawings, in which like parts are denoted by like reference numerals, and in which:-
Figure 1 is a view of a person wearing one example of a leg bag according to the invention;
Figure 2 is a view of an example of a leg bag holder;
Figure 3 is a view of a probe which may constitute the lower end of a urine drainage tube, whose upper end is connected to an incontinence appliance;
Figure 4 is a front view, partly cut away, of one example of leg bag according to the invention;
Figure 5 is a front view showing the leg bag with the probe inserted;
Figure 6 is a front view, with part of the holder cut away, of the leg bag-holder combination, showing the attachment device with its movable attachment portion in its open condition;
Figure 7 is a view similar to Figure 6 but showing the attachment device closed;
Figure 8 is a side view of part of a second embodiment according to the invention, showing a clamp-probe device having a pair of foldable "wings";
Figures 9 and 9A are respectively end view and a section on the plane X-X of Figure 8 of the clamp-probe device;
Figure 10 is a side elevation showing the device in its open position;
Figure 11 shows the clamp in the closed condition ;
Figure 12 is a view at 90° to the view of Figure 11 but showing the wings in the open position; and
Figure 13 illustrates the clamp-probe device closed onto a bag for containing liquid and securely connecting the tubular probe with the bag, so that the tube cannot be accidentally pulled out of the bag.

In this specification the words "upper" and "lower" are used in a descriptive and not a limiting sense and refer to the position of parts on the assumption that the wearer of the leg bag is standing upright with his/her leg that is wearing the bag-holder combination also upright. "Forward" and "rear" used herein are used in the sense that "rear" is the closer to, and "forward" is further from, the body of the wearer.

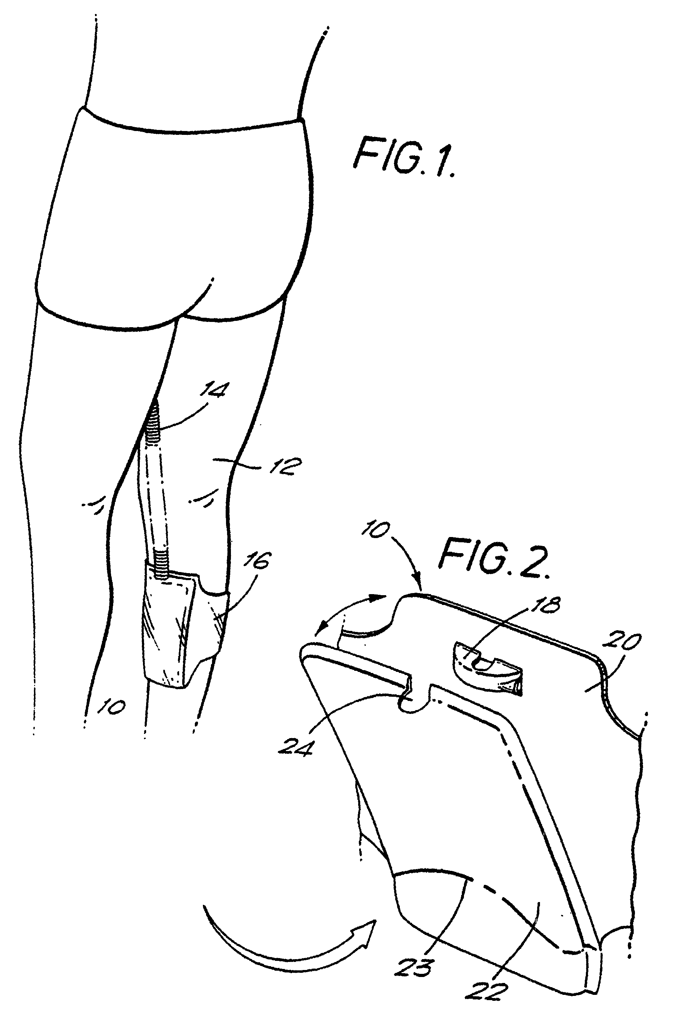

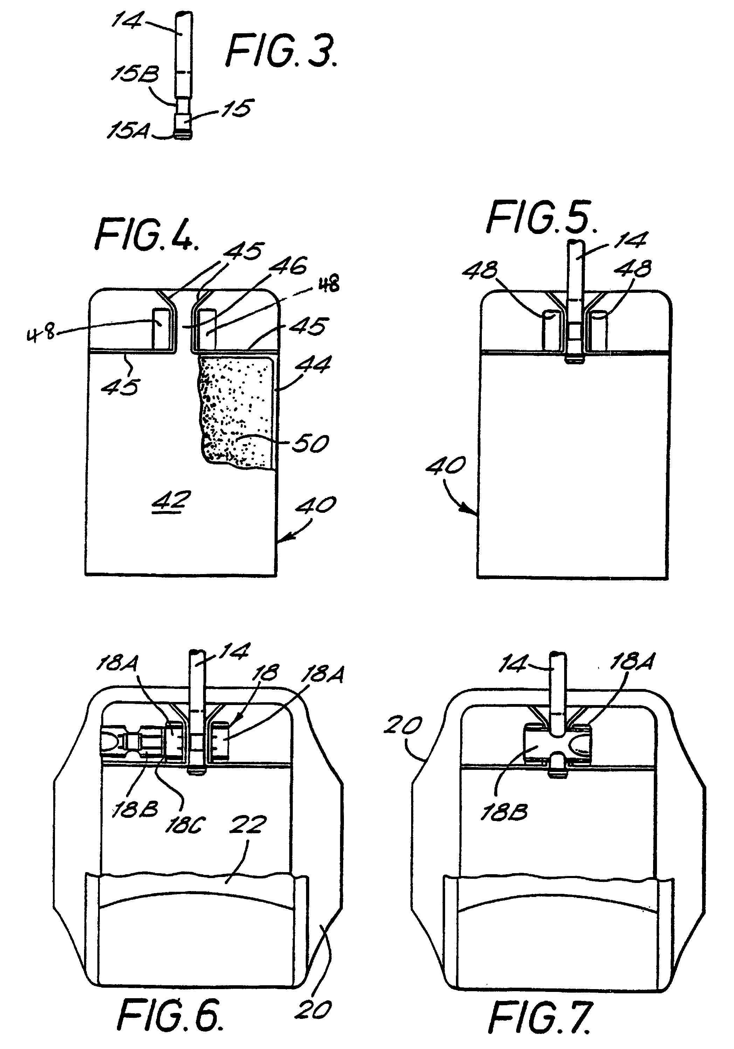

Figure 1 illustrates a holder 10, with a leg bag therein, attached to a leg 12 of the wearer. A drain tube 14, connected at its upper end to an incontinence appliance, not shown, is shown inserted into a hole in a drainage bag 40 (Fig.4). The holder 10 has a band 16 which may be fastened around the leg 12 in any convenient way (e.g. buckle and strap, VELCRO [Reg. T.M.] or buttons).

Figure 2 illustrates one form of holder according to the invention, which has a rear sheet 20 to which is hingeably connected a front sheet 22. As illustrated, this enables the front sheet 22 to pivot about a substantially horizontal axis relative to the rear sheet 20, the rear sheet 20 being connected to or integral with both the band 16 and the front sheet 22. However, as illustrated by the arrow at the lower part of the drawing, the front sheet 22 may instead be hinged about a substantially vertical axis. The front sheet 22 has a cut out 24 to accommodate the probe end of the tube 14, this being seen in Figure 3 and, in its inserted position, in Figure 5. As seen in Figure 3, the lower end of the tube 14 receives a probe 15, which may be made of plastics material, and which has a slightly enlarged and tapered end 15A. This can be forced into a hole in the upper end of the bag, as will be later described.

Referring now to Figures 4 and 5, the illustrated leg bag is made of a pair of superposed sheets (walls) of suitable plastics material, peripherally welded at 44 around the major portion of their periphery. The welds joining the front and rear walls continue inwardly and then upwardly as seen at 45. Two holes 48 are punched in part of each plastics sheet, the lower part of one such sheet being shown at 42 in Figure 4. The bag 40 contains a highly absorbent pad 50, preferably of a so-called "super-absorbent" material. Such materials are known, and those for example described in European Patent Application No. 33235A or U.S. Patent No. 4,605,401 may be employed.

The front and rear sheets 20, 22 of the holder 10 are preferably made of a flexible textile material, and a possible configuration of bending of this material is illustrated at 23 in Figure 2.

Figure 5 illustrates the tube 14 and probe 15 inserted in the hole 46 in the bag, and, in use, this bag-tube assembly is then inserted into the holder as seen in Figure 6.

An attachment device 18, comprises a fixed portion 18A and a movable portion 18B, suitably joined together for hinging action. The hinge, which may be a plastics "living hinge" is seen at 18C. For brevity hereafter, the attachment device 18 is referred to as a clamp. As seen in Figure 6, the clamp is in its open condition, and as seen in Figure 7, it is in its closed condition. The portion 18B has been folded over so that a cut out portion 18D thereof accommodates the diameter of the narrowed portion 15B of the probe 15, so holding the probe and in consequence the lower end of the tube securely in position even though longitudinal forces may be applied to the tube as would occur when the wearer makes vigorous movements.

The use of the device will be apparent from the following explanation.

Firstly the tube 14 and probe 15 are inserted in the hole 46 in the bag 40. Then the bag 40 is placed within the holder 20 with the clamp moveable portion 18B threaded through the left hand hole 48 and a shallowly upstanding portion 18A as seen in Figure 6 extending through the right hand hole 48. Then the clamp is closed by pivoting its movable portion 18B so that it overlies the probe 15, resulting in the assembly seen in Figure 7, in which it will be seen that the bag 40 is securely and comfortably retained in the holder 10. When it is desired to empty the bag, or fit a new bag, the clamp 18 is opened, the probe 15 is withdrawn from the hole 46 and the bag 40 can then if desired be discarded.

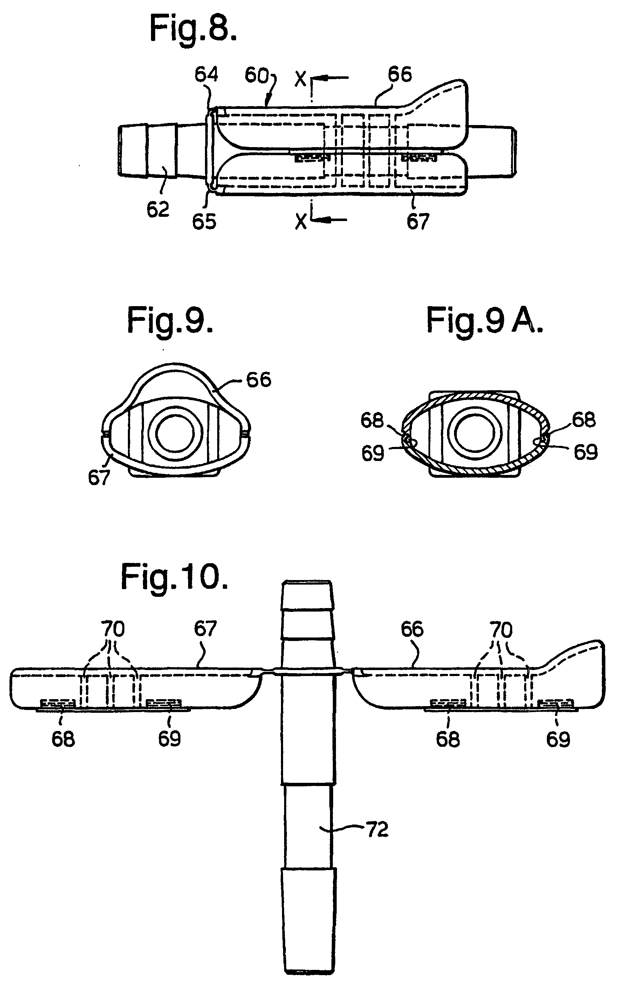

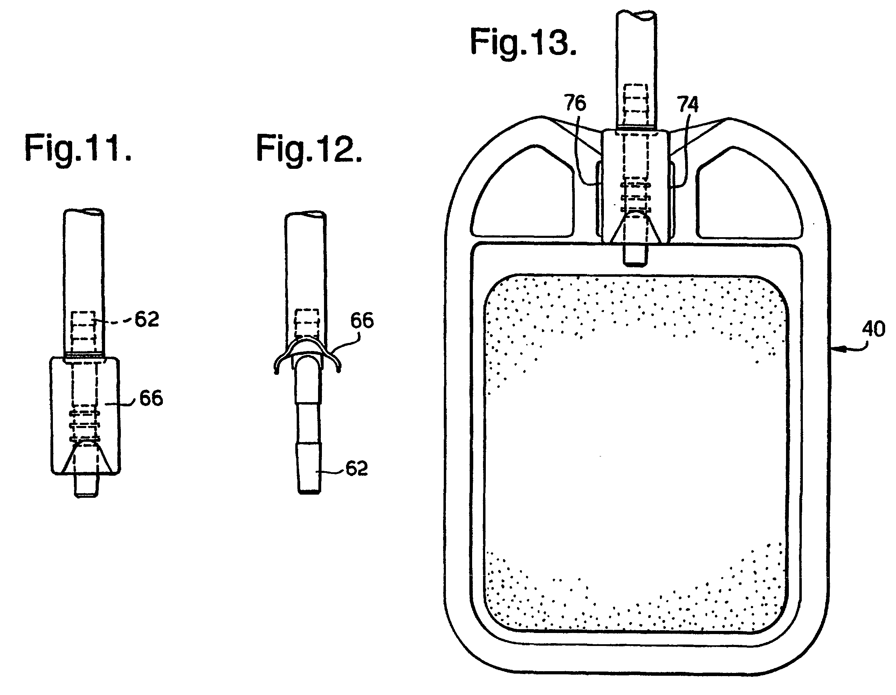

Referring now to Figures 8-13, the illustrated clamp-probe device 60 is made of a mouldable plastics, e.g. polypropylene and comprises a hollow tubular portion 62 to which is integrally attached, by so-called "plastics hinges" 64 and 65, a pair of wings 66 and 67. The shape of the wings can be seen from Figures 8-10. It will be noted that there is an engaging detent and recess structure 68,69 on each wing so that when folded closed they are releasably connected together. The inside surface of each wing has a number of shallow projections 70 which when the wings are in their closed position are received in a recess 72 formed in and extending around the external surface of the tubular portion 60. In use, the clamp device-probe 60,62 can be opened by lifting each wing 66 or 67, and the probe is then inserted into a suitable entry passageway provided at the top of the bag 40 for containing liquid. The clamp is then closed by manually swinging each wing 66,67 through substantially 90° and then squeezing the two wings together so that the structures 68,69 interengage. The edges of these structures are able to interengage by virtue of the presence of holes 74,76, Figure 13, in the upper part of the bag, which are the equivalent of the holes at 48 in Figure 5 in the first embodiment of the invention.

Important advantages this embodiment of the invention are that the clamp-probe can very easily be inserted into a drainage bag, a secure attachment is achieved by virtue of the wings, and yet disengagement is easy, even for relatively non-dexterous users, when it is desired to disconnect the tube from the bag.

Among the advantages of the invention are that it is unobtrusive and comfortable to wear, and that it provides a very secure attachment between the tube 14 and the bag. Due to this construction, the occurrence of leaks is minimized. According to an advantageous embodiment of the invention, the leg bag 40 which will be discarded, can be made from a disposable material which dissolves or disintegrates when flushed down a water-closet (w.c.).

## Claims

1. A combination comprising a bag (40) for containing liquid, a holder (10) wearable by a user, an inflow tube probe (15; 60) and an attachment device (18; 66, 67), wherein:
the holder (10) comprises a first sheet (20) of material which can conform to the curvature of a portion of the body of the wearer, a second sheet (22) of material attached to the first sheet for movement relative to the first sheet generally about an axis;
the bag (40) has an entry hole (46) at its upper end into which the inflow tube probe (15; 60) is receivable and first and second through holes (48; 74, 76) through which at least a portion of the attachment device may pass;
**characterised in that** the attachment device (18; 66, 67) is connected to the first sheet (20) or to the inflow tube probe (60), and consists of a clamp (18A, 18B; 66, 67) which when open permits the tube probe (15, 60) to be inserted in or separated from the entry hole (46) of the bag, and when closed and engaged with the through holes (48; 74, 76) secures the tube probe (15, 60) relative to the bag (40) to prevent removal of the tube probe.

2. A combination according to claim 1, wherein the clamp (18A, 18B) is carried by the first sheet (20) of the holder (10), and when the clamp is closed it secures the inflow tube probe (15) and the bag (40) to the holder (10).

3. A combination according to claim 2, wherein the attachment device (66, 67) is carried by the tube probe (60) and comprises first and second pivotable clamping wings (66, 67) attached to the tube probe (60).

4. A combination according to any preceding claim, wherein the tube probe (15, 60) comprises a recessed portion (15B; 72) to accommodate the attachment device when in its closed position.

5. A combination according to any preceding claim, wherein the second sheet (22) is pivotable about a substantially horizontal axis located at or near the bottom of the first sheet (20).

6. A combination according to any of claims 1 to 4, wherein the second sheet (22) is hinged, door-like, about a substantially vertical axis.

7. A combination according to any preceding claim, wherein the holder (10) is capable of being worn on the wearer's leg, and the bag (40) is a leg bag for carrying on the leg.

8. A combination according to any preceding claim, wherein the bag contains a pad (50) of highly absorbent material.

## Patentansprüche

1. Kombination mit einem Beutel (40) zur Aufnahme einer Flüssigkeit, einer durch einen Benutzer tragbaren Halterung (10), einer Einlaufrohrsonde (15; 60) und einer Befestigungsvorrichtung (18; 66, 67), worin
die Halterung (10) eine erste Folie (20) aus Material, die sich an die Krümmung eines Körperabschnitts des Trägers anpassen kann, und eine zweite Folie (22) aus Material, die so an der ersten Folie angebracht ist, dass sie sich allgemein um eine Achse herum relativ zur ersten Folie bewegen kann, umfasst;
der Beutel (40) an seinem oberen Ende eine Eintrittsöffhung (46), die die Einlaufrohrsonde (15; 60) aufnehmen kann, sowie eine erste und zweite Passage (48; 74, 76), durch die zumindest ein Teil der Befestigungsvorrichtung hindurchgehen kann, besitzt;
**dadurch gekennzeichnet, dass** die Befestigungsvorrichtung (18; 66, 67) mit der ersten Folie (20) oder mit der Einlaufrohrsonde (60) verbunden ist und aus einer Klammer (18A, 18B; 66, 67) besteht, die, wenn sie offen ist, es gestattet, die Rohrsonde (15, 60) in die Eintrittsöffnung (46) des Beutels einzusetzen oder daraus zu entfernen, und die, wenn sie geschlossen ist und sich im Eingriff mit den Passagen (48; 74, 76) befindet, die Rohrsonde (15, 60) bezüglich des Beutels (40) festhält, um ein Entfernen der Rohrsonde zu verhindern.

2. Kombination gemäss Anspruch 1, worin die Klammer (18A, 18B) von der ersten Folie (20) der Halterung (10) getragen wird und, wenn sie geschlossen ist, die Einlaufrohrsonde (15) und den Beutel an der Halterung (10) festhält.

3. Kombination gemäss Anspruch 2, worin die Befestigungsvorrichtung (66, 67) von der Rohrsonde (60) getragen wird und einen ersten und zweiten, schwenkbaren Klemmflügel (66, 67) umfasst, die an der Rohrsonde (60) angebracht sind.

4. Kombination gemäss einem beliebigen vorangehenden Anspruch, worin die Rohrsonde (15, 60) einen eingeschnürten Abschnitt (15B; 72) umfasst, um die Befestigungsvorrichtung aufzunehmen, wenn diese sich in ihrer geschlossenen Stellung befindet.

5. Kombination gemäss einem beliebigen vorangehenden Anspruch, worin die zweite Folie (22) um eine im Wesentlichen waagerechte, an oder nahe dem unteren Ende der ersten Folie (20) befindliche Achse schwenkbar ist.

6. Kombination gemäss einem beliebigen der Ansprüche 1 bis 4, worin die zweite Folie (22) türartig um eine im Wesentlichen senkrechte Achse schwenkbar eingehängt ist.

7. Kombination gemäss einem beliebigen vorangehenden Anspruch, worin die Halterung (10) am Bein des Trägers getragen werden kann und der Beutel (40) ein Beinbeutel zur Mitführung am Bein ist.

8. Kombination gemäss einem beliebigen vorangehenden Anspruch, worin der Beutel ein Kissen (50) aus hochsaugfähigem Material enthält..

## Revendications

1. Ensemble comprenant une poche (40) destinée à contenir du liquide, un support (10) pouvant être porté par un utilisateur, une sonde tubulaire d'écoulement (15 ; 60), et un dispositif de fixation (18 ; 66, 67), dans lequel :
le support (10) comprend une première feuille (20) de matériau qui peut se conformer à la courbure d'une partie du corps du porteur, une seconde feuille de matériau (22) fixée à la première feuille pour un mouvement relatif par rapport à la première feuille généralement autour d'un axe ;
la poche (40) comporte un trou d'entrée (46) au niveau de son extrémité supérieure dans lequel la sonde tubulaire d'écoulement (15 ; 60) peut être reçue, et des premier et second trous traversants (48 ; 74, 76) au travers desquels peut passer au moins une partie du dispositif de fixation ;
**caractérisé en ce que** le dispositif de fixation (18 ; 66, 67) est relié à la première feuille (20) ou à la sonde tubulaire d'écoulement (60), et est constitué d'une attache (18A, 18B, 66, 67) qui, lorsqu'elle est ouverte, permet que la sonde tubulaire (15, 60) soit insérée dans ou maintenue à l'écart du trou d'entrée (46) de la poche, et, lorsqu'elle est fermée et en prise avec les trous traversants (48 ; 74, 76) elle fixe la sonde tubulaire (15, 60) par rapport à la poche (40) afin d'empêcher un retrait de la sonde tubulaire.

2. Ensemble selon la revendication 1, dans lequel l'attache (18A, 18B) est supportée par la première feuille (20) du support (10), et lorsque l'attache est fermée elle fixe la sonde tubulaire d'écoulement (15) et la poche (40) au support (10).

3. Ensemble selon la revendication 2, dans lequel le dispositif de fixation (66, 67) est supporté par la sonde tubulaire (60) et comprend des première et seconde ailes de fixation pivotantes (66, 67) fixées à la sonde tubulaire (60).

4. Ensemble selon l'une quelconque des revendications précédentes, dans lequel la sonde tubulaire (15, 60) comprend une partie d'étranglement (15B ; 72) afin de loger le dispositif de fixation lorsqu'il se trouve dans sa position fermée.

5. Ensemble selon l'une quelconque des revendications précédentes, dans lequel la seconde feuille (22) peut pivoter autour d'un axe sensiblement horizontal situé au niveau du bas de la première feuille ou à proximité de celui-ci (20).

6. Ensemble selon l'une quelconque des revendications 1 à 4, dans lequel la seconde feuille (22) est articulée, à la façon d'une porte, autour d'un axe sensiblement vertical.

7. Ensemble selon l'une quelconque des revendications précédentes, dans lequel le support (10) peut être porté sur la jambe d'un porteur, et la poche (40) est une poche en jambière destinée à être portée sur la jambe.

8. Ensemble selon l'une quelconque des revendications précédentes, dans lequel la poche contient un tampon (50) réalisé en matériau hautement absorbant.
